# EUROPÄISCHE PATENTSCHRIFT

(11) **EP 1 210 906 B1**
(45) Veröffentlichungstag und Bekanntmachung des Hinweises auf die Patenterteilung: **07.06.2006**
(21) Anmeldenummer: 01128066.6
(22) Anmeldetag: 26.11.2001
(51) Int. Cl.: A61B 5/00

(54) **Vorrichtung zur Bestimmung von Gewebeperfusion**
Apparatus for determining tissue perfusion
Dispositif pour déterminer l'état de perfusion tissulaire

(30) Priorität: 28.11.2000 DE 10059070
(43) Veröffentlichungstag der Anmeldung: 05.06.2002
(73) Patentinhaber: Pulsion Medical Systems AG, 81829 München (DE)
(72) Erfinder: Pfeiffer, Ulrich, J., Dr., 81667 München (DE); Becker, Andreas, Dr., 85570 Markt Schwaben (DE); Eder, Norbert, 84543 Winhöring (DE); Burger, Thorsten, Dr., 80804 München (DE); Töns, Christian, Dr., 47447 Moers (DE)
(74) Vertreter: Kehl, Günther

(56) Entgegenhaltungen:
- US-A- 5 363 854
- US-A- 6 032 070
- US-A- 6 081 612
- STILL J., LAW E., DAWSON J,: "Evaluation of the circulation of reconstructive flaps using induced fluorescence of indocyanine green" ANNALS OF PLASTIC SURGERY, Bd. 42, Nr. 3, März 1999 (1999-03), Seiten 266-274, XP001063252
- PATENT ABSTRACTS OF JAPAN vol. 1998, no. 13, 30. November 1998 (1998-11-30) & JP 10 201707 A (OLYMPUS OPTICAL CO LTD), 4. August 1998 (1998-08-04)

## Beschreibung

Die vorliegende Erfindung betrifft eine Vorrichtung zur Bestimmung von Gewebeperfusion bei Anwendungen, bei denen das Tragen von Schutzbrillen eine Behinderung darstellen würde, und die intraoperative Verwendung einer solchen Vorrichtung.

In "Microvascular Research" 1994, Vol. 47, S. 240-251 sind eine Vorrichtung und und ein Verfahren zur Angiofluorographie der Haut beschrieben. Dabei wird nach Applikation von Indocyaningrün die Durchblutung der Haut anhand des Fluoreszenzsignals beurteilt, das durch Anregung mittels Bestrahlung im nahinfraroten Bereich entsteht. Als Strahlungsquelle dient hierbei eine 2000 Watt Halogenlampe mit vorgeschaltetem Bandpassfilter sowie einem aufgrund der starken Wärmeentwicklung erforderlichen Wasserfilter. Als Detektor dient eine CCD-Kamera.

In US 5,074,306 sind eine Vorrichtung und ein Verfahren zur Untersuchung von Verbrennungen mittels Fluoreszenzmessungen nach Applikation eines exogenen Chromophors offenbart. Die Bildgebungsapparatur besteht aus einer Xenon- oder Quecksilberdampf-Lampe als Anregungslichtquelle, einer intensivierten CCD-Kamera und einem Computer mit Bildbearbeitungssoftware.

Werden Halogen- oder Quecksilberdampflampen als Lichtquellen für die Anregung des Chromophors verwendet, so müssen diese eine relativ hohe Leistung aufweisen, da nur ein geringer Wellenlängenbereich der breitbandigen Abstrahlung zur Fluoreszenzanregung nutzbar ist, und das übrige erzeugte Licht mittels eines Bandpassfilters herausgefiltert werden muß. Die hohe erforderliche Lampenleistung im Kilowatt-Bereich führt zu entsprechend großer Wärmeerzeugung, welche Kühlmaßnahmen erforderlich macht, sowie zu einer Baugröße insbesondere der elektrischen Komponenten. Tragbare, netzunabhängige Ausführungen sind daher mit derartigen Lichtquellen nicht möglich.

Aus "Annals of Plastic Surgery" 1999, Vol. 42, S. 266-274 sind eine Vorrichtung und ein Verfahren zur Messung der Durchblutung von transferierten Hautlappen bekannt, wobei nach Applikation von Indocyaningrün die durch Bestrahlung mit einem gepulsten Laser-Array angeregte Fluoreszenz mit einer CCD-Kamera detektiert wird.

In "Photochemistry and Photobiology" 1999, 70(1), S. 87-94 sind eine Vorrichtung und ein Verfahren zur Tumorerkennung in tierischem Gewebe beschrieben, wobei nach Applikation von Indocyaningrün die durch Bestrahlung mit einer Laser-Diode angeregte Fluoreszenz mit einer CCD-Kamera detektiert wird.

Der Einsatz entsprechender Laser-Lichtquellen erfordert aus Sicherheitsgründen das Tragen von Schutzbrillen bei der Handhabung der Systeme sowie die Kennzeichnung der Räume, in denen sie angewendet werden, durch Warnschilder. Dies stellt für rein diagnostische Aufgaben kein wesentliches Problem dar, macht derartige Systeme jedoch ungeeignet für die intraoperative Anwendung, da sich besondere Laserschutzmaßnahmen im chirurgischen Routineeinsatz störend auswirken würden.

In US 5,400,791 ist eine Vorrichtung für die Infrarot-Videoangiographie des Augenhintergrunds offenbart, die eine Nahinfrarot-Lichtquelle zur Anregung der Fluoreszenz von Indocyaningrün sowie eine Detektorkamera aufweist.

Die in US 5,400,791 offenbarte Vorrichtung eignet sich jedoch aufgrund Ihrer Auslegung für die okularmedizinische Diagnostik nicht zur intraoperativen Betrachtung eines größerflächigen Operationsfeldes.

Aufgrund der mangelnden Eignung herkömmlicher auf der Fluoreszenzanregung eines Chromophors beruhender diagnostischer Vorrichtungen für die routinemäßige Anwendung im Operationssaal liegt der vorliegenden Erfindung die Aufgabe zugrunde, eine Vorrichtung zur intraoperativen Bestimmung von Gewebeperfusion zu schaffen, die bequem handhabbar ist, sich auch für nicht-mikroinvasive Eingriffe eignet und die insbesondere spezielle Schutzvorkehrungen, wie etwa das Tragen von Schutzbrillen, überflüssig macht.

Diese Aufgabe wird durch eine Vorrichtung zur Bestimmung von Gewebeperfusion gemäß Anspruch 1 gelöst, die eine Strahlungsquelle, welche ein Strahlbündel in einem Wellenlängenbereich erzeugt, der dem Wellenlängenbereich der Fluoreszenzanregung des Chromophors Indocyaningrün entspricht, und eine Aufweitungsoptik aufweist, ferner eine Sicherheitseinhausung und eine elektronische Kamera mit einem optischen Filter, der durchlässig für emittiertes Licht des angeregten Chromophors und undurchlässig für den Wellenlängenbereich des erzeugten Laserstrahls ist. Dabei sind die Strahlungsquelle und die Aufweitungsoptik so in die Sicherheitseinhausung integriert, daß nur ein aufgeweitetes Strahlbündel aus der Sicherheitseinhausung austreten kann, dessen Intensität einen die Gefährdung ohne Schutzbrillen in der Nähe der Vorrichtung sich aufhaltender Personen ausschließenden Grenzwert nicht übersteigt, und die Aufweitungsoptik ermöglicht die Ausleuchtung eines auch für nicht mikroinvasive Eingriffe hinreichend großen Operationsfeldes. Somit eignet sich die Erfindung zum Einsatz insbesondere dort, wo die Quantifizierung der Gewebedurchblutung während eines Eingriffs entscheidend sein kann, d.h. im Bereich der Viszeralchirurgie bei Linkskolon- und Rektumresektionen, bei Schluchmagentransposition nach Ösophagusresektion, bei freien Dünndarmtransplantaten zur Interposition sowie allen Roux-Y Rekonstruktionen (nach gastrektomie, als biliodigestive Anastomosen etc.) Auch eignet sich die Vorrichtung zur Erkennung sekundärer Perfusionsstörungen bei inkarzerierter Hernie oder bei Bridenileus. In der Herzchirurgie kann die Vorrichtung zur Überprüfung der Effizienz koronarer Bypässe eingesetzt werden. Im Bereich der plastischen Chirurgie ist die Kontrolle der Perfusion transferierter Lappen, sowie die Beurteilung des Gewebeschadens bei Traumata (z.B. Navikularfrakturen, Mehrfragmentfrakturen, Weichteilverletzungen sowie Schußverletzungen) möglich.

Erfindungsgemäß handelt es sich bei der Strahlungsquelle um eine Infrarot-Laser-Lichtquelle mit einer Peakemission im Wellenlängenbereich zwischen 750 nm und 810 nm, vorzugsweise 780 nm.

Der die Gefährdung ohne Schutzbrillen in der Nähe der Vorrichtung sich aufhaltender Personen ausschließende Grenzwert kann sich vorzugsweise aus der Europäischen Norm EN 60825-1 ableiten. Für den nah-infraroten Bereich errechnet sich hier die maximal zulässige Bestrahlung (MZB) für die Einwirkung von Laserstrahlung auf die Hornhaut des Auges bei Einwirkungsdauern in der für die medizische Anwendung der vorliegenden Erfindung relevanten Größenordnung nach der Formel MZB = 18·t^{0,75} ·C₄·C₆ J·m⁻², worin t für die Einwirkungsdauer steht und sich die Parameter C₄ und C₆ wie folgt ergeben: Für eine Peakwellenlänge λ der Infrarot-Laser-Lichtquelle zwischen 700 und 1050 nm ist C₄ = 10^{0,002·((λ/ₙₘ-700))}. Ist die Winkelausdehnung α der scheinbaren Quelle (d.h. des aufgeweiteten Laserstrahls beim Austritt aus der Sicherheitseinhausung) vom Auge des Betrachters aus in einem Abstand von mindestens 100 mm gemessen größer als αₘₐₓ=100 mrad und die Einwirkungsdauer größer oder gleich 10 Sekunden, so ist C₆ = αₘₐₓ/ αₘᵢₙ =100mrad/11mrad = 9,09 (mit αₘᵢₙ =11 mrad). Folglich muß bei einer Einwirkungsdauer über zehn Sekunden der die Gefährdung in der Nähe der Vorrichtung sich aufhaltender Personen ausschließende Grenzwert für die Intensität des aus der Sicherheitseinhausung austretenden aufgeweiteten Laserstrahls kleiner als der Ausdruck 9,2·10^{0,002·((λ/ₙₘ-700))}mW·cm⁻² sein, womit der Grenzwert für das bevorzugte Anwendungsgebiet unterhalb etwa 13,4 mw/cm² liegt.

Die Vorrichtung weist erfindungsgemäß ferner ein elektronisches Bildauswertesystem auf, welches zur Bestimmung der Helligkeit der Pixel in ausgewählten Bildbereichen ausgestattet ist.

In einer besonders bevorzugten Ausführungsform weist die Infrarot-Laser-Lichtquelle mindestens eine Laserdiode auf, besonders geeignet ist eine Anzahl von 1 bis 10 Dioden, deren Leistung im Bereich von unter 1 mW bis 1 W, vorzugsweise zwischen unter 1 W und 300 mW liegt.

Vorteilhafterweise kann die Infrarot-Laser-Lichtquelle auch gepulst oder moduliert sein.

Vorzugsweise weist die Aufweitungsoptik mindestens eine Streulinse und mindestens eine Sammellinse auf.

Vorteilhafterweise kann ein Element der Aufweitungsoptik, vorzugsweise eine der Linsen, mattiert sein.

Die Divergenz des aus der Sicherheitseinhausung austretenden aufgeweiteten Strahlbündels beträgt vorzugsweise zwischen 0° und 40°.

Zur Erfassung einer hinreichend großen Fläche ermöglicht die Aufweitungsoptik die Bestrahlung eines vorzugsweise 10 bis 40 cm breiten Bereichs in einem Abstand von vorzugsweise 20 bis 200 cm von der Sicherheitseinhausung.

In einer besonders vorteilhaften Weiterbildung weist die Vorrichtung einen

Akkumulator auf und kann somit netzunabhängig betrieben werden. Der Wegfall eines Netzkabels vermeidet potentielle Einschränkungen der Bewegungsfreiheit des Operateurs und der anderen an einem Eingriff Beteiligten.

In einer besonders bevorzugten Ausführungsform ist die elektronische Kamera, bei der es sich um eine im Consumerbereich übliche CCD-Videokamera handeln kann, einhändig tragbar und bedienbar.

In einer vorteilhaften Weiterbildung sind die Sicherheitseinhausung und die elektronische Kamera zu einer kompakten Einheit zusammengefaßt.

In einer vorteilhaften Ausführungsform besitzt die Vorrichtung eine Schnittstelle zur, vorzugsweise digitalen, Bildübertragung, wobei die Bildübertragung auch drahtlos realisiert werden kann.

In einer weiteren vorteilhaften Ausführungsform ist die Vorrichtung fernsteuerbar und beispielsweise an der Decke über dem Operationstisch montiert. Somit kann die Bedienung durch eine Person erfolgen, die nicht direkt an der Operation beteiligt ist, so daß die Beeinträchtigung des Operationsablaufs durch die Messung minimal ist.

Vorzugsweise weist die Kamera einen Sucher oder einen integrierten LCD-Bildschirm auf, so daß ein externer Bildschirm entfallen kann, dessen Platzbedarf und erforderliche Kabelverbindung zur Kamera die Bewegungsfreiheit des Operateurs und der anderen an einem Eingriff Beteiligten einschränken könnte.

Vorzugsweise ist die Vorrichtung mit einem Speichermittel, beispielsweise in Form von Speicherchips oder einer Videokassette, ausgestattet, um Bilddaten aufzeichnen zu können.

Nachstehend wird ein Ausführungsbeispiel der vorliegenden Erfindung anhand der schematischen, nicht maßstäblichen Zeichnungen erläutert. Hierbei zeigt:
- Fig.1:: die schematische Anordnung einer erfindungsgemäßen Vorrichtung bei der intraoperativen Verwendung,
- Fig.2:: den schematischen Aufbau der Sicherheitseinhausung der Vorrichtung aus Fig.1 mit integrierter Laserdiode und Strahlaufweitungsoptik.

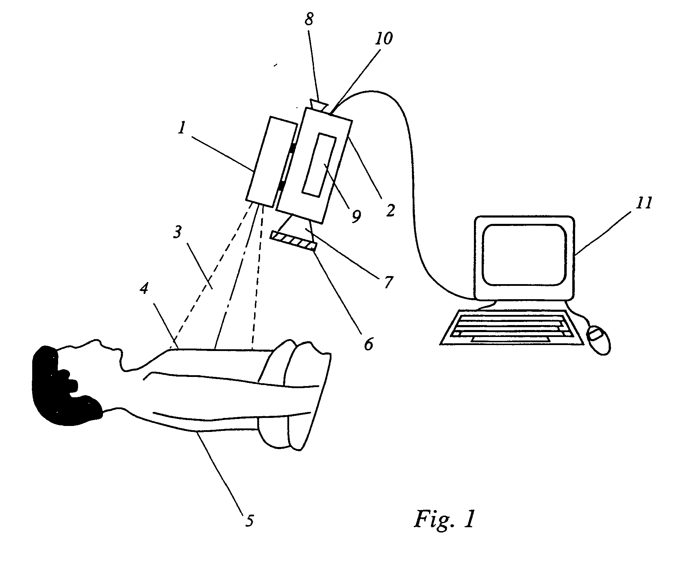

In Fig.1 ist schematisch und nicht maßstäblich die Anordnung einer erfindungsgemäßen Vorrichtung im intraoperativen Einsatz dargestellt. Die Sicherheitseinhausung 1, in welche die Infrarot-Laser-Lichtquelle mit einer Peakemission von 780 nm integriert ist, bildet zusammen mit der CCD-Kamera 2 eine kompakte, einhändig tragbare und bedienbare Einheit, die über einen Akkumulator verfügt und daher netzunabhängig einsetzbar ist.

Das aus der Sicherheitseinhausung 1 austretende aufgeweitete Laserlicht 3 hat eine flächenbezogene Intensität von unter 1 mW/cm² und liegt somit unter dem Grenzwert der maximal zulässigen Bestrahlung der Hornhaut des Auges (MZB), wodurch in der Umgebung der Vorrichtung keine Schutzbrillen getragen werden müssen.

Das aufgeweitete Strahlbündel 3 der Infrarot-Laser-Lichtquelle bestrahlt das etwa 30 cm breite Operationsfeld 4, das sich in etwa 70 cm Entfernung von der Sicherheitseinhausung 1 befindet. Dem Patienten 5 zuvor intravenös als Bolus in einer Dosis zwischen 0,1 und 2 mg pro kg Körpergewicht injiziertes Indocyanin wird durch die Bestrahlung zur Fluoreszenz angeregt.

Das Fluoreszenzsignal wird von der im nahinfraroten Wellenlängenbereich empfindlichen CCD-Kamera 2 detektiert, der ein Filter 6 vorgeschaltet ist. Der Filter 6 ist ein NIR-Langpassfilter (Kantenfilter), der nur für Wellenlängen größer 800 nm durchlässig ist und mittels eines Außengewindes mit dem Autofokus-Objektiv 7 der CCD-Kamera 2 verschraubt ist. Alternativ eignet sich auch ein Filter, der eine schmalbandige Transmission im Bereich des Fluoreszenzmaximums des Chromophors Indocyaningrün erlaubt. Die CCD-Kamera 2 besitzt einen Sucher 8, so daß während der Operation kein externer Monitor eingesetzt werden muß, und somit eine möglicherweise die Handhabung beeinträchtigende Kabelverbindung entfällt. Die elektronischen Bilddaten der aufgenommenen Fluoreszenz werden auf einer Videokassette 9 digital aufgezeichnet.

Zwischen dem Patienten 5 und der Einheit aus CCD-Kamera 2 und Sicherheitseinhausung 1 kann ein steriles Tuch (nicht dargestellt) angeordnet werden, so daß die Vorrichtung selbst nicht steril sein muß. Aufgrund der kompakten Bauweise und des Wegfallens von Kabelverbindungen kann die Einheit aus CCD-Kamera 2 und Sicherheitseinhausung 1 aber auch leicht steril verpackt werden.

Über eine Schnittstelle 10 nach IEEE 1394, die eine Datenübertragungsrate von bis zu 400 MBit/s erlaubt, kann ein elektronisches Bildverarbeitungs- und -auswertesystem 11 an die CCD-Kamera 2 angeschlossen werden. die es erlaubt die Helligkeit der einzelnen Bildelemente (Pixel) quantitativ als Maß für die Fluoreszenzintensität zu erfassen. Hierzu können auf dem ersten Bild einer Bildsequenz durch den Benutzer verschiedene Bildbereiche (Regions of interest) markiert werden, um dann Bild für Bild die Helligkeit der Pixel in diesem Areal zu ermitteln und die Ergebnisse grafisch darzustellen. Dabei kann ein zu untersuchendes Gewebeareal direkt mit einem Referenzareal mit normaler Perfusion oder aber mit einem externen Standard (z.B. einer Fluoreszenzfolie) bekannter Intensität verglichen werden. Bei der Verwendung eines externen Standards können auch Bildsequenzen, die mit unterschiedlichen Bestrahlungs- oder Detektorparametern aufgenommen wurden, direkt miteinander verglichen werden. Durch die Auswertung der gesamten Bildsequenz ist es möglich, verschiedene Kriterien, wie z. B. die Geschwindigkeit der Anflutung und Abflutung des Chromophors und die durch das Chromophor verursachte Veränderung der Fluoreszenzintensität in den Gewebearealen zur Auswertung heranzuziehen.

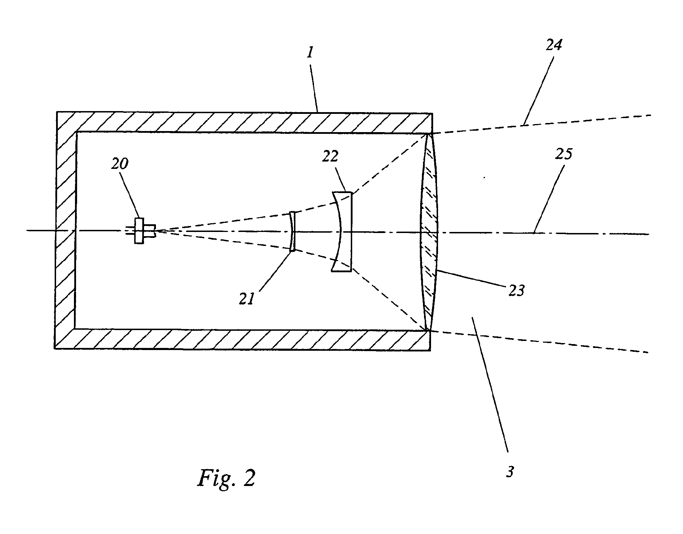

In Fig.2 ist schematisch der Aufbau der Sicherheitseinhausung 1 der Vorrichtung aus Fig.1 mit integrierter Laserdiode 20 und Strahlaufweitungsoptik gezeigt, die aus den Streulinsen 21 und 22 und der mattierten Sammellinse 23 besteht. Das aus der Sicherheitseinhausung austretende Strahlbündel 3 weist eine Divergenz von 10° (entsprechend einem Winkel von 5° zwischen Randstrahl 24 und optischer Achse 25) auf. Durch die Aufweitungsoptik ist gewährleistet, daß aus der Sicherheitseinhausung 1 keine Strahlung austritt, die eine Gefährdung der in der Nähe der Vorrichtung sich aufhaltenden Personen auftritt, ohne daß hierfür besondere zusätzliche Schutzmaßnahmen, wie etwa das Tragen von Schutzbrillen, ergriffen werden müßten.

## Patentansprüche

1. Vorrichtung zur Bestimmung von Gewebeperfusion bei Anwendungen, bei denen das Tragen von Schutzbrillen eine Behinderung darstellen würde, welche folgendes aufweist:
eine Infrarot-Laser-Lichtquelle (20), die ein elektromagnetisches Strahlbündel in einem Wellenlängenbereich erzeugt, der dem Wellenlängenbereich der Fluoreszenzanregung von Indocyaningrün entspricht,
eine Aufweitungsoptik,
eine Sicherheitseinhausung (1),
eine elektronische Kamera (2) mit einem optischen Filter (6), der durchlässig für emittiertes Licht des angeregten Indocyaningrüns und undurchlässig für den Wellenlängenbereich des erzeugten Strahlbündels ist,
und ein elektronisches Bildauswertesystem (11), welches zur Bestimmung der Helligkeit der Pixel in ausgewählten Bildbereichen ausgestattet ist,
wobei die Lichtquelle und die Aufweitungsoptik so in die Sicherheitseinhausung (1) integriert sind, daß nur ein aufgeweitetes Strahlbündel (3) aus der Sicherheitseinhausung (1) austreten kann, dessen Intensität den durch den Ausdruck 9,2·10^{0,002·((λ/ₙₘ-700))}mW·cm⁻² gegebenen Grenzwert nicht übersteigt, wenn mit λ die Peakwellenlänge der Infrarot-Laser-Lichtquelle (20) bezeichnet ist,und die Aufweitungsoptik die Ausleuchtung eines auch für nicht mikroinvasive Eingriffe hinreichend großen Operationsfeldes (4) ermöglicht.

2. Vorrichtung nach Anspruch 1, **dadurch gekennzeichnet, daß** die Infrarot-Laser-Lichtquelle (20) mindestens eine Laserdiode aufweist.

3. Vorrichtung nach einem der vorangehenden Ansprüche, **dadurch gekennzeichnet, daß** die Infrarot-Laser-Lichtquelle (20) gepulst ist.

4. Vorrichtung nach einem der vorangehenden Ansprüche, **dadurch gekennzeichnet, daß** die Infrarot-Laser-Lichtquelle (20) moduliert ist.

5. Vorrichtung nach einem der vorangehenden Ansprüche, **dadurch gekennzeichnet, daß** die Aufweitungsoptik mindestens eine Streulinse (21, 22) und mindestens eine Sammellinse (23) aufweist.

6. Vorrichtung nach einem der vorangehenden Ansprüche, **dadurch gekennzeichnet, daß** die Aufweitungsoptik ein mattiertes Element aufweist.

7. Vorrichtung nach Anspruch 6, **dadurch gekennzeichnet, daß** das mattierte Element eine mattierte Linse (23) ist.

8. Vorrichtung nach einem der vorangehenden Ansprüche, **dadurch gekennzeichnet, daß** das aus der Sicherheitseinhausung (1) austretende Strahlbündel (3) eine Divergenz von 0° bis 40° aufweist.

9. Vorrichtung nach einem der vorangehenden Ansprüche, **dadurch gekennzeichnet, daß** die Aufweitungsoptik die Bestrahlung eines 10 bis 40 cm breiten Bereichs (4) in einem Abstand von 20 bis 200 cm von der Sicherheitseinhausung (1) ermöglicht.

10. Vorrichtung nach einem der vorangehenden Ansprüche, **dadurch gekennzeichnet, daß** die Vorrichtung einen Akkumulator aufweist und netzunabhängig betrieben werden kann.

11. Vorrichtung nach einem der vorangehenden Ansprüche, **dadurch gekennzeichnet, daß** die elektronische Kamera (2) einhändig tragbar und bedienbar ist

12. Vorrichtung nach einem der vorangehenden Ansprüche, **dadurch gekennzeichnet, daß** die Sicherheitseinhausung (1) und die elektronische Kamera (2) zu einer kompakten Einheit zusammengefaßt sind.

13. Vorrichtung nach einem der vorangehenden Ansprüche, **dadurch gekennzeichnet, daß** die Vorrichtung eine Schnittstelle (10) zur Bildübertragung aufweist.

14. Vorrichtung nach Anspruch 13, **dadurch gekennzeichnet, daß** die Schnittstelle (10) zur Bildübertragung eine Schnittstelle zur digitalen Bildübertragung ist.

15. Vorrichtung nach einem der vorangehenden Ansprüche, **dadurch gekennzeichnet, daß** die Vorrichtung fernsteuerbar ist.

16. Vorrichtung nach einem der vorangehenden Ansprüche, **dadurch gekennzeichnet, daß** die Kamera einen Sucher (8) aufweist.

17. Vorrichtung nach einem der vorangehenden Ansprüche, **dadurch gekennzeichnet, daß** die Vorrichtung einen integrierten LCD-Bildschirm aufweist.

18. Vorrichtung nach einem der vorangehenden Ansprüche, **dadurch gekennzeichnet, daß** die Vorrichtung mindestens ein Speichermittel (9) zur Speicherung von Bilddaten aufweist.

## Claims

1. A device for the determination of tissue perfusion in applications in which the wearing of safety glasses would constitute a hindrance, said device comprising the following:
an infra-red laser light source (20), generating an electromagnetic bundle of rays in a wavelength range corresponding to the wavelength range of the fluorescence excitation of indocyanin green,
an optical expansion unit,
a safety housing (1),
an electronic camera (2) with an optical filter (6), which is transparent to emitted light of the excited indocyanin green and opaque to the wavelength range of said generated bundle of rays, and an electronic image evaluation system (11), which is provided for the determination of the brightness of the pixels in selected regions of the image,
wherein the light source and the optical expansion unit are integrated into the safety housing (1) so that there can only emerge from the safe housing (1) an expanded bundle of rays (3) with an intensity not exceeding a limit value that is given by the expression 9.2·10^{0.002((λ/nm-700))}mW·cm⁻², wherein λ designates the peak wavelength of the infrared laser light source (20), and the optical expansion unit enables the illumination of an operating area (4) sufficiently large even for non-microinvasive procedures.

2. A device according to claim 1, **characterised in that** the infrared laser light source (20) comprises at least one laser diode.

3. A device according to any of the preceding claims, **characterised in that** the infrared laser light source (20) is pulsed.

4. A device according to any of the preceding claims, **characterised in that** the infrared laser light source (20) is modulated.

5. A device according to any of the preceding claims, **characterised in that** the optical expansion unit comprises at least one diffusing lens (21,22) and at least one collecting lens (23).

6. A device according to any of the preceding claims, **characterised in that** the optical expansion unit has an obscure element.

7. A device according to Claim 6, **characterised in that** the wherein the obscure element is a frosted lens (23).

8. A device according to any of the preceding claims, **characterised in that** the bundle of rays (3) emerging from the safety housing (1) has a divergence of 0° to 40°.

9. A device according to any of the preceding claims, **characterised in that** the optical expansion unit enables the radiation of a 10 to 40 cm wide area (4) at a distance of 20 to 200 cm from the safety housing (1).

10. A device according to any of the preceding claims, **characterised in that** the arrangement comprises an accumulator and can be operated independently of the mains.

11. A device according to any of the preceding claims, **characterised in that** the electronic camera (2) can be carried and operated with one hand.

12. A device according to any of the preceding claims, **characterised in that** the safety housing (1) and the electronic camera (2) are combined to form a compact unit.

13. A device according to any of the preceding claims, **characterised in that** the device comprises an interface (10) for image transmission.

14. A device according to Claim 13, **characterised in that** the interface (10) for image transmission is an interface for digital image transmission.

15. A device according to any of the preceding claims, **characterised in that** the device can be remote-controlled.

16. A device according to any of the preceding claims, **characterised in that** the camera has a viewfinder.

17. A device according to any of the preceding claims, **characterised in that** the device comprises an integrated LCD screen.

18. A device according to any of the preceding claims, **characterised in that** the device comprises at least one storage means (9) for storing image data.

## Revendications

1. Dispositif pour la détermination de la perfusion tissulaire lors d'utilisations dans lesquelles le port de lunettes protectrices représenterait une gêne, lequel présente ce qui suit :
- une source de lumière laser infrarouge (20), qui produit un faisceau de rayonnement électromagnétique dans une plage de longueurs d'onde qui correspond à la plage de longueurs d'onde de l'excitation de fluorescence du vert d'indocyanine ;
- une optique d'élargissement ;
- une installation de sécurité (1);
- une caméra électronique (2) avec un filtre optique (6), qui est perméable à la lumière émise du vert d'indocyanine excité et opaque à la plage de longueurs d'onde du faisceau de rayonnement produit ; et
- un système électronique (11) d'évaluation d'images, lequel est équipé pour la détermination de la luminosité des pixels dans des zones d'images choisies,
la source de lumière et l'optique d'élargissement étant intégrées dans le boîtier de sécurité (1) de telle sorte que seul un faisceau de rayonnement élargi (3) peut sortir du boîtier de sécurité (1), dont l'intensité ne dépasse pas la valeur limite donnée par l'expression 9,2.10 ^{0,002-((?/nm-700))} mW.cm⁻², lorsque par ? on désigne la longueur d'onde de pic de la source de lumière laser infrarouge (20), et l'optique d'élargissement permet l'illumination d'un champ d'opération (4) suffisamment important également pour des opérations non micro-invasives.

2. Dispositif selon la revendication 1, **caractérisé par le fait que** la source de lumière laser infrarouge (20) présente au moins une diode laser.

3. Dispositif selon l'une des revendications précédentes, **caractérisé par le fait que** la source de lumière laser infrarouge (20) est pulsée.

4. Dispositif selon l'une des revendications précédentes, **caractérisé par le fait que** la source de lumière laser infrarouge (20) est modulée.

5. Dispositif selon l'une des revendications précédentes, **caractérisé par le fait que** l'optique d'élargissement présente au moins une lentille divergente (21, 22) et au moins une lentille convergente (23).

6. Dispositif selon l'une des revendications précédentes, **caractérisé par le fait que** l'optique d'élargissement présente un élément dépoli.

7. Dispositif selon la revendication 6, **caractérisé par le fait que** l'élément dépoli est une lentille dépolie (23).

8. Dispositif selon l'une des revendications précédentes, **caractérisé par le fait que** le faisceau de rayonnement sortant de l'installation de sécurité (1) présente une divergence de 0° à 40°.

9. Dispositif selon l'une des revendications précédentes, **caractérisé par le fait que** l'optique d'élargissement permet l'irradiation d'une zone (4) de 10 à 40 cm de large à une distance de 20 à 200 cm de l'installation de sécurité (1).

10. Dispositif selon l'une des revendications précédentes, **caractérisé par le fait que** le dispositif présente un accumulateur et peut être actionné indépendamment du réseau.

11. Dispositif selon l'une des revendications précédentes, **caractérisé par le fait que** la caméra électronique (2) est portable et utilisable d'une main.

12. Dispositif selon l'une des revendications précédentes, **caractérisé par le fait que** l'installation de sécurité (1) et la caméra électronique (2) sont réunies en une unité compacte.

13. Dispositif selon l'une des revendications précédentes, **caractérisé par le fait que** le dispositif présente une interface (10) pour la transmission d'images.

14. Dispositif selon la revendication 13, **caractérisé par le fait que** l'interface (10) pour la transmission d'images est une interface pour la transmission numérique d'images.

15. Dispositif selon l'une des revendications précédentes, **caractérisé par le fait que** le dispositif est commandable à distance.

16. Dispositif selon l'une des revendications précédentes, **caractérisé par le fait que** la caméra présente un viseur (8).

17. Dispositif selon l'une des revendications précédentes, **caractérisé par le fait que** le dispositif présente un écran à cristaux liquides (LCD) intégré.

18. Dispositif selon l'une des revendications précédentes, **caractérisé par le fait que** le dispositif présente au moins une mémoire (9) pour le stockage de données d'images.
